# EUROPEAN PATENT SPECIFICATION

(11) **EP 3 409 253 B1**
(45) Date of publication and mention of the grant of the patent: **28.10.2020**
(21) Application number: 17173913.9
(22) Date of filing: 01.06.2017
(51) Int. Cl.: A61F 13/62, A44B 18/00, A61F 13/56

(54) **A FASTENING TAB WITH ANTI-FLAGGING FEATURE**
BEFESTIGUNGSLASCHE MIT ANTI-MARKIERUNGSMERKMAL
LANGUETTE DE FIXATION DISPOSANT D'UNE FONCTION ANTI-SIGNALEMENT

(43) Date of publication of application: 05.12.2018
(73) Proprietor: 3M Innovative Properties Company, Saint Paul, MN 55133-3427 (US)
(72) Inventor: Güler, Serhat, 59860 Çorlu-Tekirda (TR)
(74) Representative: Vollmers, Hans-Gerd

(56) References cited:
- EP-A1- 1 543 807
- EP-A1- 2 965 735
- US-A1- 2007 049 890

## Description

### Field

The present disclosure relates to fastening tabs for attachment to articles, for example fastening tabs for disposable articles, for example diapers. The present disclosure furthermore relates to tapes of fastening tabs and to articles comprising the fastening tabs. Moreover, the present disclosure relates to a method of making fastening tabs and a method of applying fastening tabs to an article.

### Background

Fastening tabs are widely used in mechanical closure systems, for example in closure systems of articles, typically mass-produced articles, such as diapers. The fastening tabs comprise a substrate, typically a backing layer, and have an end region for attaching the tab to the article, e.g., to the outer side of a diaper's ear. The attachment of this end of the tap to the article is usually done by the manufacturer of the article. This end of the tab is also referred to in the art as 'manufacturer's end'. The opposite end of the tabs contains a region with fastening elements. This end is gripped by the user of the article to attach it to a receiving surface to close the article. For example, in case the article is a diaper, the manufacturer's end of the tab is attached to one ear of the diaper and the other end of the tab will be gripped and attached to the other ear of the diaper to fix the diaper around the wearer's waist. Therefore, the end of the tab containing the fastening elements is also referred to in the art as 'user's end'. The fastening elements typically comprise hooks that can releasably engage a loop material provided on the surface of the diaper, typically provided as a so-called 'landing zone' on the diaper.

EP 1 543 807 A1 relates to a closure tape tab for an absorbent article, particularly for a disposable diaper, for fastening the article on the body of a person. The closure tape tab comprises a proximal end portion and a distal end portion being connected by an inner tab portion, wherein the inner tab portion has a first major surface and a second major surface. The proximal and distal end portions are connected to the inner tab portion at the first major surface thereof such that opposing ends of the proximal and distal end portions are spaced apart from each other. An anti-adhesive means is provided at at least a part of the first major surface of the inner tab portion in the space.

During production and storage of the articles it is generally undesirable that the user's end of the fastening tab freely extends from the article. This may cause interruption of the production lines or may damage the article or the tab during mass production of the articles. Therefore, the user's end of the fastening tab is often folded around the surface to which it is attached by its manufacturer's end, e.g. the outer side of a diaper's ear, and temporarily attached to the other side of the surface, e.g. the inner side of said diaper's ear. The tab is kept in this position until the article is used. This technique is referred to as "anti-flagging." Anti-flagging can be provided in several ways. Exposed areas of adhesive in the fastening region of the tab may hold the user's end down on the article and keep the tab in an anti-flagging position. Examples of such tabs are described, for instance, in WO 2005/000180 A1 and WO 2012/112768 A1. However, using adhesives in the fastening region may lead to damage of the loop material on of the article. Repeated opening and closing of the fastening tab, for example for readjusting the diaper on the wearer's waist may lead to poorer engagement and poorer closing performance of the fastening tab and the closure tab may pop open on its own. In WO 2015/153327 an adhesive of low or now tackiness is provided in the fastening region and a tackier adhesive is provided in proximity to the fastening to provide anti-flagging.

An alternative approach to provide anti-flagging employs the use of release tapes that bond to the article. This approach has been termed 'Y-bonding' and is described, for example, in WO 2015/066210 A1.

However, there is a need for alternative tab constructions with anti-flagging properties. The constructions provided herein use a cover tape to provide anti-flagging properties.

### Summary

Therefore in one embodiment there is provided a fastening tab for an article comprising a substrate having a first end region with a first outermost end and opposite thereto a second end region with a second outermost end, and a middle region between the first and second end regions, wherein the first end region comprises at least one fastening region comprising a fastening material attached to the substrate wherein the fastening material comprises a plurality of mechanical fastening elements for releasable fastening, the tab further comprising a cover tape covering at least a portion of the middle region and wherein at least a first portion of the cover tape is connected to the middle region by at least one releasable connection, wherein the at least one releasable connection comprises a releasable, non-adhesive bond. The cover tape is connected by a second portion to the substrate by at least one non-releasable connection, wherein the at least one non-releasable connection is situated between the at least one releasable connection and the second end region. The cover tape comprises a surface that can be attached to the article to create a bond between the article and that surface of the cover tape. The non-adhesive bond is a created by ultrasonic welding and wherein the tape and the substrate comprise, at least at their releasable connection, a thermoplastic material that does not differ in melting point or differs in melting point by less than 315,15 °K (42°C).

In another embodiment there is provided a tape comprising a plurality of the fastening tabs, wherein the fastening tabs are arranged either individually or in endless form on the tape in a cross section so that individual tabs can be cut from the fastening tape.

In a further embodiment there is provided an article comprising the fastening tab wherein the tab is connected to a surface of the article by the second end portion of the tab.

In yet another embodiment there is provided Method of making a fastening tab the method comprising the steps of
a) providing the cover tape and the substrate having the first end region with a first outermost end and opposite thereto the second end region with a second outermost end, and the middle region between the first and second regions, wherein the first end region comprises at least one fastening region comprising a fastening material attached to the substrate wherein the fastening material comprises a plurality of mechanical fastening elements for releasable fastening;
b) connecting the cover tape at least by its first portion to the middle region of the substrate by at least one releasable connection made by ultrasonic welding such that the tape covers at least a portion of the middle region of the substrate, wherein the at least one releasable connection comprises a releasable, non-adhesive bond.

In another aspect there is provided a method of applying the fastening tab to an article, the method comprising
i) providing the fastening tab;
ii) attaching the fastening tab with its second end region to a surface of the article.

Fastening tabs for attachment to an article are provided may contain no or only low amounts of exposed adhesives. The fastening tabs can be brought into an anti-flagging position on the article. Further embodiments are also described in the dependent claims.

In the following, terms such as "a", "an" and "the" are not intended to refer to only a singular entity, but include the general class of which a specific example may be used for illustration. The terms "a", "an", and "the" are used interchangeably with the term "at least one". The phrases "at least one of" and "comprises at least one of' followed by a list refers to any one of the items in the list and any combination of two or more items in the list. All numerical ranges are inclusive of their endpoints and non-integral values between the endpoints unless otherwise stated.

The terms "first" and "second" are used in this disclosure. It will be understood that, unless otherwise noted, those terms are used in their relative sense only. For these components, the designation of "first" and "second" may be applied to directions, features, or components merely as a matter of convenience in the description of one or more of the embodiments.

The terms "multiple" and "a plurality" refer to more than one.

The above summary of the present disclosure is not intended to describe each disclosed embodiment or every implementation of the present disclosure. The description that follows more particularly exemplifies illustrative embodiments. It is to be understood, therefore, that the drawings and following description are for illustration purposes only and should not be read in a manner that would unduly limit the scope of this disclosure.

### Brief Description of Figures

Figure 1A shows a schematic representation of a fastening tab according to the present disclosure in cross-sectional view.
Figure 1B shows a schematic representation of a fastening tab according to the present disclosure in cross-section sectional view where the fastening tab is connected by its second end region to a surface of an article.
Figure 1C shows a schematic representation of a fastening tab according to the present disclosure shown in cross-sectional view where the fastening tab is attached and folded around the surface of the article in the anti-flagging position.
Figure 1D shows a schematic representation of a fastening tab according to the present disclosure in cross-sectional view after the tap has been opened from its anti-flagging position.
Figure 2A shows a schematic representation of another embodiment of a fastening tab according to the present disclosure in cross-sectional view.
Figure 2B shows a schematic representation of another embodiment of a fastening tab according to the present disclosure shown in cross-section where the fastening tab is connected by its second end region to a part of an article.
Figure 2C shows a schematic representation of another embodiment of a fastening tab according to the present disclosure shown in cross-sectional view where the fastening tab is attached and folded around a part of an article in the anti-flagging position.
Figure 2D shows a schematic representation of another embodiment of a fastening tab according to the present disclosure in cross-sectional view after the tab has been opened from its anti-flagging position.
Figure 3 shows a schematic representation in perspective view of a roll of a fastening tabs tape according to the present disclosure.
Figure 4 shows a schematic representation in perspective view of a diaper containing fastening tabs according to the present disclosure.
Figure 5A shows a schematic representation of another embodiment of a fastening tab according to the present disclosure in cross-sectional view.
Figure 5B shows a schematic representation of another embodiment of a fastening tab according to the present disclosure shown in cross-section where the fastening tab is connected by its second end region to a part of an article.
Figure 5C shows a schematic representation of another embodiment of a fastening tab according to the present disclosure shown in cross-sectional view where the fastening tab is attached and folded around a part of an article in the anti-flagging position.
Figure 5D shows a schematic representation of another embodiment of a fastening tab according to the present disclosure in cross-sectional view after the tab has been opened from its anti-flagging position.

### Detailed Description

The tabs according to the present description will now be described in greater detail. It will be referred to the figures but referring to the figures is done for illustration only. It will be understood that this disclosure is not meant to be limited to the specific embodiments and their structures, composition and configurations illustrated in these figures.

In the figures the following reference numerals are used:
Surface of an article (ear of a diaper) = 1;
first surface of article (e.g. outer surface of the ear of a diaper) = 5;
second (opposite) surface of the article (e.g. inner surface of that ear of a diaper) = 6;
fastening tab = 10;
fingerlift = 12;
substrate (backing) = 13;
cover tape = 15;
first outermost end of tab = 16;
second outermost end of tab = 17;
bond between second end region and surface of article = 18;
bond of cover tape to article = 19;
blanking film = 20;
second connection (non-releasable connection) = 21;
second connection (non-releasable connection) via folded tape = 21'
releasable connection in the form of a non-adhesive, releasable bond 22;
fastening material = 31;
first end region (user's end) = 41;
middle region = 42;
second end region = 43;
roll of a fastening tabs tape = 100;
diaper = 111;
loop material on a landing zone of the diaper = 4;
liquid permeable top sheet of a diaper = 2;
liquid impermeable backsheet of a diaper = 3;
rear waist region of diaper = 7;
front waist region of diaper = 8.

The fastening tabs according to the present disclosure comprise a backing layer or substrate, a first end region (the user's end) and opposite thereto a second end region, the manufacturer's end. Between first and second end regions there is a middle region. The first end region comprises the first outermost end of the tab and the second end region comprises the second outermost end of the tab. The second end region is the manufacturer's end of the tab and will be used for permanent attachment of the tab to an article. The tabs may be provided without adhesives on the second end. Adhesives may be applied to the second ends before or during attachment of the tabs to the article, or the tabs may be attached to the articles by an attachment that does not employ adhesives, for example but not limited to attachments created by lamination, ultrasonic welding, stitching. The first end region comprises the fastening material with a plurality of mechanical fastening elements for releasable engagement with a receiving surface on the article.

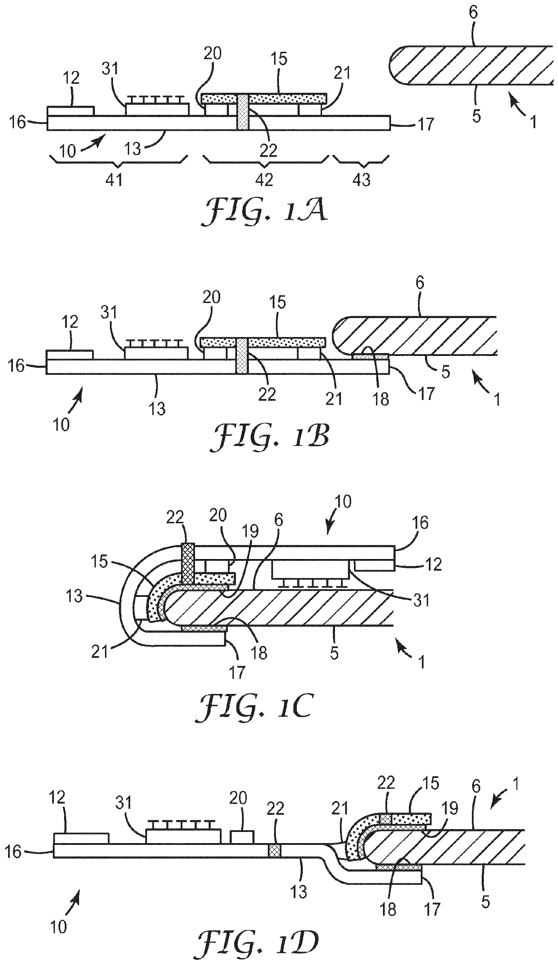

Referring to FIG. 1, 2 and 5 there are shown schematic representations of some different embodiments of fastening tabs according to the present disclosure viewed in cross-section and in various stages of their attachment to an article. FIG. 1A, 2A and 5A show the fastening tabs in a flat configuration, i. e. before they are attached to an article. FIG. 1B-1D, 2B-2D and 5B-5D show the application of the tabs to a surface of an article (here an ear of a diaper). FIG 1B, 2B and 5B show the attachment of the tabs via their second end regions to the outer surface of a diaper's ear. FIG.1C, 2C and 5C show the tabs in the anti-flagging position attached to the opposite surface of the diaper's ear after the fastening tabs have been folded around the edge of the diaper's ear. FIG. 1D, 2D and 5D show the opening of the tabs from their anti-flagging position so that they can be applied to a receiving zone on the article, for example the backsheet of a diaper, for closing the diaper around the wearer's waist.

The substrate of the tabs according to the present disclosure (represented by layer 13 in the figures) may be a backing layer as is typically used in the art of making closure tabs. For example, the substrate may be selected from a variety of films or sheetings including single-layered and multi-layered films, coextended films, laterally laminated films or films comprising film layers. The layers of such films or sheetings may comprise various materials such as, for example, polypropylene, polyvinylchloride, polyethylene terephthalate, polyethylene, polyolefin copolymers or blends of polyolefins such as, for example, a blend of polypropylene, low density polyethylene and/or linear low density polyethylene, textiles, and non-woven and foamed materials. The thickness of the substrate is preferably between 30 microns and 500 microns, and more preferably between 40 and 150 microns. The base weight of the substrate is preferably between 15 and 500 g/m², more preferably between 20 and 300 g/m² and particularly preferably between 20 and 200 g/m².

The first end region of the tabs of the present disclosure (represented by region 41 in the figures) comprises a fastening region containing the fastening material for releasable fastening of the tab to the article. The fastening material (represented as 31 in the figures) comprises a plurality of fastening elements. The first end region extends from the first outermost end (represented by end 16) of the tap over the fastening material and to the end of the fastening material. The fastening region may extend over the entire first end region or may only cover a part of the first end region leaving an area extending from the outmost end 16 uncovered by fastening material, for example, to allow for the presence of a finger lift and/or an area of exposed adhesive. The latter is not necessary and may even be undesired and the area may simply comprise exposed substrate. In a particular embodiment of the present disclosure the first end region contains a fingerlift and an exposed area of substrate between fingerlift and fastening region. In a preferred version of that embodiment, the area between fingerlift and fastening region is free of exposed adhesive, and may be, for example, an area of exposed substrate.

The embodiment illustrated in FIG. 1 contains a fingerlift 12 placed at the outermost end 16 and an area uncovered by fingerlift 12 and fastening material 31 situated between fingerlift 12 and fastening material 31. A fingerlift facilitates grasping the first end region by a user. The fingerlift may be a different material than the substrate -as is illustrated in FIG 1. In this case the fingerlift 12 may be connected to the substrate by an adhesive bond or a non-adhesive bond. However, it is also possible to provide a substrate structured such it forms a fingerlift and substrate and fingerlift are an integral structure of the same material. In this case, the substrate may have a raised area that can function as a fingerlift. Typically, the fingerlift may have a structured surface that facilitates gripping of the first end region.

The first end region of the tabs according to the present disclosure contains a fastening region containing the fastening material. The fastening material of the tabs according to the present disclosure contains a plurality of fastening elements. The fastening elements preferably are mechanical elements. They typically include male elements that can releasably engage (entangle) with a corresponding receiving material, for example a loop material. The entangled fastening elements provide appropriate resistance against peel forces and shear forces such that a closure of the article, e.g. a diaper is provided (at least after a first or second engagement). Typically, the entanglement is strong enough to provide a closure but still allows for releasing the tab from the article by manually pulling the tab away from the article. The strength of the engagement depends on the intended use of the tab. For example, in case of diapers, the entanglement is strong enough to keep the diaper closed but the closure can be opened by manually pulling the firs end region of the fastening tab away from the receiving surface of the article.

The fastening material of the fastening tabs according to the present disclosure may be made of a thermoplastic material. Suitable thermoplastic materials include polyolefin homopolymers such as polyethylene and polypropylene, copolymers of ethylene, propylene and/or butylene; copolymers containing ethylene such as ethylene vinyl acetate and ethylene acrylic acid; polyesters such as poly(ethylene terephthalate), polyethylene butyrate and polyethylene napthalate; polyamides such as poly(hexamethylene adipamide); polyurethanes; polycarbonates; poly(vinyl alcohol); ketones such as polyetheretherketone; polyphenylene sulfide; and mixtures thereof. Typically, in embodiments in which fastening material contains integral male fastening elements, the thermoplastic is a polyolefin (e.g., polyethylene, polypropylene, polybutylene, ethylene copolymers, propylene copolymers, butylene copolymers, and copolymers and blends of these materials). In some embodiments where the fastening material include integral fastening elements the fastening material contains a backing from which the fastening elements extend. The backing and the male fastening elements are typically integral (that is, formed at the same time as a unit, unitary). Upstanding fastening elements on a backing can be made, for example, by feeding a thermoplastic material onto a continuously moving mold surface with cavities having the inverse shape of the fastening elements of their precursors, for example, posts which may be later shaped to create the final male fastening elements. The thermoplastic material can be passed between a nip formed by two rolls or a nip between a die face and roll surface, with at least one of the rolls having the cavities. The cavities may be in the inverse shape of a capped post having a loop- engaging head or may be in the inverse shape of an upstanding post without loop-engaging heads (e.g., a precursor to a male fastening element). Pressure provided by the nip forces the resin into the cavities. In some embodiments, a vacuum can be used to evacuate the cavities for easier filling of the cavities. The nip typically has a large enough gap such that a coherent backing is formed over the cavities. The mold surface and cavities can optionally be air or water cooled before stripping the integrally formed backing and upstanding hook elements from the mold surface such as by a stripper roll. If the posts formed upon exiting the cavities do not have loop-engaging heads, loop-engaging heads could be subsequently formed into hooks by a capping method as described in U.S. Pat. No. 5,077,870 (Melbye et al.). Typically, the capping method includes deforming the tip portions of the hook elements using heat and/or pressure. The heat and pressure, if both are used, could be applied sequentially or simultaneously.

The formation of male fastening elements can also include a step in which the shape of the cap is changed, for example, as described in U.S. Pat. No. 6,132,660 (Kampfer).

Suitable tool rolls include those formed from a series of plates defining a plurality of post- forming cavities about its periphery such as those described, for example, in U.S. Pat. No. 4,775,310 (Fischer). Cavities may be formed in the plates by drilling or photoresist technology, for example. Other suitable tool rolls may include wire-wrapped rolls, which are disclosed along with their method of manufacturing, for example, in U.S. Pat. No. 6, 190,594 (Gorman et al.). Another example of a method for forming a thermoplastic backing with upstanding posts includes using a flexible mold belt defining an array of upstanding post-shaped cavities as described in U.S. Pat. No. 7,214,334 (Jens et al.). Yet other useful methods for forming a thermoplastic backing with upstanding posts can be found in U.S. Pat. Nos. 6,287,665 (Hammer), 7, 198,743 (Tuma), and 6,627,133 (Tuma).

The male fastening elements may have loop-engaging heads that have an overhang or may be upstanding posts having distal tips that can be formed into loop-engaging heads, if desired. The term "loop-engaging" as used herein relates to the ability of a male fastening element to be mechanically attached to a loop material. Generally, male fastening elements with loop-engaging heads have a head shape that is different from the shape of the post. For example, the male fastening element may be in the shape of a mushroom (e.g., with a circular or oval head enlarged with respect to the stem), a hook, a nail, a T and combinations thereof. Typically, male fastening elements that have loop-engaging heads have a maximum thickness dimension (in either dimension normal to the height) of up to about 1 (in some embodiments, 0.9, 0.8, 0.7, 0.6, 0.5, or 0.45 or even 0.2 or 0.1) millimeter. In some embodiments, the male fastening elements have a maximum height (above the backing) of up to 3mm, 1.5 mm, 1 mm, or 0.5 mm and, in some embodiments a minimum height of at least 0.05 mm, 0.1 mm, or 0.2 mm, or even 0.1 mm. In some embodiments, the upstanding posts have aspect ratio (that is, a ratio of height to width at the widest point) of at least about 2: 1, 3: 1, or 4: 1.

In some embodiments, the thickness of the backing portion of the fastening material described herein may be up to about 400, 250, 150, 100, 75 or 50 micrometers, depending on the desired application, which does not necessarily include the height of male or female mechanical fastening elements on the surface of the backing portion of the fastening material. In some embodiments, the thickness of the backing portion of the fastening material is in a range from 30 to about 225 micrometers, from about 50 to about 200 micrometers, or from about 100 to about 150 micrometers. Specific examples of fastening elements, their shapes and dimensions and their production are also described, for example, in WO94/18864, WO97/46129, WO99/10161, WO00/06363, WO00/151808, EP 1,365,669, US 5,868957, US 5,077,820, US 5,900,350.

Surfaces for receiving the fastening elements to create a releasable engagement (receiving surfaces) include materials containing fibers. The fibers may be of sufficient size to receive the fastening materials and include materials arranged as loops. Receiving materials and loop materials useful for practicing some embodiments of the present disclosure can be any suitable material that interlocks with corresponding male fastening elements. In some embodiments, the material is typically formed from knitted fabrics, woven fabrics, or non-woven fabrics. The term "non-woven" refers to a material having a structure of individual fibers or threads that are interlaid but not in an identifiable manner such as in a knitted fabric. Examples of non-woven webs include spunbond webs, spunlaced webs, airlaid webs, meltblown web, and bonded carded webs. The spread web may include fiber loops projecting from a knitted, woven, or non-woven backing or may be extrusion-bonded, adhesive-bonded, and/or sonically-bonded fiber loops. Useful materials may be made of natural fibers (e.g., wood or cotton fibers), synthetic fibers (e.g., thermoplastic fibers), or a combination of natural and synthetic fibers. Examples of suitable materials for forming thermoplastic fibers include polyolefins (e.g., polyethylene, polypropylene, polybutylene, ethylene copolymers, propylene copolymers, butylene copolymers, and copolymers and blends of these polymers), polyesters, and polyamides. The fibers may also be multi-component fibers, for example, having a core of one thermoplastic material and a sheath of another thermoplastic material.

In some embodiments, the receiving material used for practicing some embodiments of the present disclosure comprises a loop material comprising a fibrous layer disposed on a backing. Suitable backings include textiles, paper, thermoplastic films (e.g., single- or multilayered films, coextruded films, laterally laminated films, or films comprising foam layers), and combinations thereof. For thermoplastic backings, the thermoplastic can be any of those described above in connection with a thermoplastic backing having male fastening elements.

The receiving material, including loop material, may be provided as a separate zone on the article on which the tabs are to be used or the surface of the article may be such that is contains such material with which the fastening elements can engage.

The fastening tabs according to the present disclosure may comprise the fastening material arranged as discrete patches such as discrete stripes of fastening material. In another embodiment, the fastening tab comprises one or more patches of fastening material that are interconnected, wherein the one or more patches of fastening material preferably form a reticulated mechanical fastener comprising multiple strands of fastening material attached and/or integral to each other at bridging regions of fastening material. Examples of such a reticulated mechanical fastener are described in detail in WO 2012/112768 A1.

The fastening material of the tabs according to the present disclosure may also be arranged to form a single, continuous area.

Typically, the fastening material is connected to the substrate by a non-releasable bond. A non-releasable bond is a stronger bond than a releasable bond. The non-releasable bond is stronger than the releasable engagement of the fastening elements with their corresponding receiving material. The non-releasable bond is intended to be maintained during use of the tab for attaching the tab to the article and during the intended, at least single use of the article. Therefore, such a bond is also referred to herein as a "permanent" bond. The non-releasable bond may be created by one or more adhesives or by a bond created without using any adhesives (including but not limited to heat welding, lamination or ultrasonic welding), or a combination thereof. For example the creation of permanent bonds using ultrasonic welding is described in WO2014/204829.
In one embodiment the substrate (e.g. substrate layer 13 in the figures) is used to make the fastening material and the fastening elements. This means the fastening elements protrude directly from the substrate. In this case fastening elements and substrate can form an integral structure.

In a preferred embodiment the fastening material is attached to the substrate by an adhesive.

The fastening region may comprise one or more areas not containing fastening material, ie., the fastening material may not be continuous but is discontinuous. In case one or more adhesives have been used to attach the fastening material to the substrate, such areas not covered by fastening material may contain exposed adhesive. To avoid damage of the receiving or loop material by the exposed adhesive an adhesive with low tackiness may be used, or an adhesive that becomes non-tacky after application is used preferably. Adhesives which are favorably useful for connecting the fastening material to the substrate include hot melt adhesives, in particular, fast-setting hot melt adhesives and/or hot melt adhesives which are not tacky or only slightly tacky at ambient temperatures (e. g. 296,15 °K (23 °C) and 50 % RH). An example of such a hot melt adhesive is the adhesive marketed by H.B. Fuller. St Paul, MN, USA under the trade designation LUNATACK HL 1696 X ZP L. Other adhesives which may be favorably used as adhesives include contact adhesives and latent-reactive adhesives (adhesives that are non-tacky at room temperatures but achieve a tacky state at higher temperatures, e. g. in excess of 323,15 °K (50 °C)). Latently reactive adhesive dispersions and films are for example marketed by Epurex GmbH, an associated company of Bayer MaterialScience AG, Leverkusen, Germany. Such films are composed on semicrystalline polyurethane polymer of the type marketed under the trade designation DISPERCOLL U into which surface-deactivated solid isocyanate particles are integrated, while such dispersions include a polyurethane dispersion of the aforementioned type in combination with deactivated solid isocyanate. The fastening material may be attached to the substrate via a contact adhesive by providing contact adhesive on the underlying surface of the fastening material and then bringing the two surfaces together to allow for self-adhesion of the contact adhesive and thus bonding of the fastening material to the substrate. The fastening material may be attached to the substrate via a latent-reactive adhesive for example by positioning a latent reactive adhesive film between the fastening material and the substrate and then raising the temperature to trigger melting and crosslinking of the polymer and thus bonding of the fastening material to the substrate. Pressure sensitive adhesives may also be useful as adhesives, preferably if their tackiness is low or none. The adhesive is preferably selected from a group of adhesives having (e.g. after an open time of at least 24 hours) a 90° peel adhesion to a smooth polyethylene test surface equal to or greater than 1 N/inch, more preferably equal to or greater than 1.5 N/inch, most preferably equal to or greater than 2 N/inch as measured according to ASTM D3330, method F using a roll-down weight of 5,000 g and where the polyethylene test surface has an average surface roughness value Ra of about 1.4 µm (e.g. measured using a laser profilometer and calculated in accordance with DIN 4768 and 4762). To minimize any tendency towards fibrous material damage, favorably the adhesive is selected from a group of adhesives having a 90° peel adhesion equal to or less than 10 N/inch, more desirably equal to or less than 9 N/inch, and most desirably equal to or less than 8 N/inch. Adhesives which are useful as adhesives include pressure-sensitive adhesives which are selected from a group comprising (meth)acrylate and/or natural or synthetic rubber-based pressure-sensitive adhesives. Rubber-resin additives preferably comprise in addition to the rubber materials one or more tackifying resins in order to render the rubber materials tacky. Preferred examples of rubber-based pressure-sensitive adhesives are the polystyrene-polyisoprene block copolymers tackified with synthetic polyterpene resins. Suitable acrylate-based pressure-sensitive adhesives are disclosed, for example, in US Re 24,906 and US 4,710,536. Suitable synthetic rubber based adhesives are described, for example, in US 5,019,071 and US 3,932,328. Coating weights of the adhesive may range from 5 to 100 g/m2. The adhesive may also be selected from a group of adhesives having a 90° peel adhesion to a smooth polyethylene test surface of less than 2 N/inch, in particular, less than 1.5 N/inch, more particularly less than 1 N/inch as measured according to ASTM D3330, method F using a roll-down weight of 5,000 g and where the polyethylene test surface has an average surface roughness value Rₐ of about 1.4 µm. The adhesive may have a tack value less than 0.6 N/mm², in particular equal to or less than 0.5 N/mm², and more particularly equal to or less than 0.4 N/mm², as determined in accordance with ASTM D 2979, where probe has an diameter of 0.8mm, and where the following conditions were used: separation rate 1 mm/min; dwell time 1 second; contact pressure 1950 kPa ; and temperature and relative humidity 296,15 °K (23 °C) and 50 %, respectively. The tackiness may also have a tack value equal to 0 (zero) N/mm².

The fastening tabs according to the present disclosure comprise a middle region (represented as region 42 in the figures) between the first and second end regions. The middle region may begin where the fastening material ends (the end of the fastening material that faces away from the first outermost region, i.e. the middle region does not contain fastening material) and may extend to the beginning of the second end region. The second end region is the region that will be bonded to the article and includes the second outermost end of the tab. The middle region of the tabs according to the present disclosure preferably does not contain any exposed adhesive. Preferably, at least a major portion of the middle region is free of exposed adhesive. The "major portion" as used herein means more than 50% of the surface area of the middle region. The surface area is determined by viewing the surface of the middle region from top. Differences in surface structure (e.g. raised areas) are ignored. Also gaps, cracks and openings are ignored when determining the surface area, i.e. the surface will be treated for determining the surface area as it were a continuous, entirely flat surface. It is an advantage of the fastening tabs of the present disclosure that they can be provided with a middle region having no exposed adhesive. In fact, the present disclosure even allows to provide fastening tabs without any exposed adhesives (although adhesives may still be used to attach the tabs to the article by the manufacturer of the article).

The middle region is at least partially covered by a cover tape (represented as layer 15 in the figures). The cover tape 15 of the present disclosure is releasably connected by at least a portion of the tape to the middle region of the substrate. The releasable connection comprises at least one releasable, non-adhesive bond (represented as 22 in the figures). The non-adhesive bond is a bond created without using an adhesive (although additional releasable bonds made with adhesives may be present but are not necessary and are preferably absent). Methods of creating a non-adhesive bond include ultrasonic welding. The at least one releasable, non-adhesive bond comprises or is a bond created by ultrasonic welding. Ultrasonic welding is known with one of the earliest references being British patent No. 1,018,971. Commercial welding machines with commercial horns and anvils may be used to practise embodiments of the present disclosure, for example an ultrasonic welding machine from Telsonic Ultrasonics, Bronschhofen, Switzerland. Ultrasonic welding uses low amplitude, high frequency vibration to create friction between the parts to be joined to generate enough heat to melt the material in the interface. When the vibration stops the materials in the interface solidify as they cool thus forming a bond between them. The frequencies used are above the range of human hearing (ultrasonic frequencies). The strength of a given weld joint depends on the amount of energy applied to the materials to be joint. Therefore, the bond strength is controlled by controlling the energy applied to the joint area during welding. The energy is controlled such that a releasable bond is created. A too high energy will lead to bond that is too strong and thus no longer releasable. The appropriate energy will depend on the materials chosen and can be determined by routine optimization. A releasable bond may also be created by generating welding dots rather than generating a continuous weld seam. The smaller the welding dots and the wider they are spaced apart the weaker and thus more releasable is the bond created by ultrasonic welding.

The releasable connection is created by ultrasonic welding, for example in the form of a plurality of ultrasonic welding dots that spaced apart from each other. In case cover tape and substrate are connected directly, which is preferred, both cover tape and substrate comprise, at least at their connection, materials that are welded together by ultrasonic welding are thermoplastic materials having melting point, e. g. a difference in melting points of less than 315,15 °K (42 °C), preferably less than 309,15 °K (36 °C). The substrate and cover tape each comprise at least one layer of a thermoplastic material, either the same thermoplastic material or thermoplastic materials differing in melting points by less than 315,15 °K (42 °C), preferably less than 309,15 °K (36 °C). The cover tape will comprise that thermoplastic layer at its bottom surface and the substrate at its top surface in case one of them or both are multi-layered materials. Cover tape and substrate are then at least in portions joined by their top and bottom surfaces, respectively.

It is also contemplated that the releasable, non-adhesive connection between cover tape and substrate is an indirect connection. This means the cover tape and substrate may be connected via one or more intermediate spacer materials and the spacer material contains the releasable, non-adhesive bond, for example at one of its interfaces with the substrate or the cover tape. For example, the cover tape may be connected to a spacer substrate by a releasable and non-adhesive bond, and the spacer substrate is connected to the substrate by a permanent bond, i.e. a stronger bond, such that the connection between cover tape and spacer substrate breaks but the connection between spacer substrate and substrate does not. Or conversely, the connection between cover tape and spacer substrate is permanent but the connection between spacer substrate and substrate is a releasable, non-adhesive bond. In this case the releasable connection breaks between substrate and spacer substrate.

This releasable, non-adhesive connection of the cover tape to the substrate (bond 22 in the figures) is weaker than the permanent bonds between fastening material and substrate. The bond 22 is also weaker than the bond by which the tab is attached to the article via its second end region, such that the cover tape can be released from the tab at least by its first portion when pulling the tab away from its anti-flagging position while the cover tape remains attached to the article by its second end region.

In a particular embodiment the surface of the middle region covered by the cover tape is free of any exposed adhesive or at least a major portion of the surface of the middle region covered by the cover tape is free of any exposed adhesive. In one embodiment the entire middle region is covered by the cover tape.

The cover tape comprises a surface that can be attached to the article to create a bond between article and that surface of the cover tape. The bond may be created between the cover tape and the article when the tab is applied to the article and put in the anti-flagging position (represented as layer 18 in figures 1, 2 and 5). The bond is stronger than the releasable bond 22 between cover tape and substrate. It may not be as strong than the permanent bonds between fastening material and substrate or between second end region and the article, or it may be of equal strength or even greater strength. To create such a bond an adhesive may be applied to that surface of the cover tape before or after the second end of the tab is attached to the article. Any suitable adhesive or mixtures of adhesives known in the art may be used. The adhesive may also be applied to that surface of the cover tape at the same time when the tab is attached to the article by its second end region. Through the bond between cover tape and article and the releasable connection of the cover tape to the substrate the tab is held down onto the article in an anti-flagging position. When opening the tab from the anti-flagging position, for example, by gripping the tab by its first end region and pulling it away from the surface of the article, the releasable connection between tab and cover tape (represented as 22 in the figures) breaks and the cover tape remains bonded to the article. Therefore, in a preferred embodiment, no exposed adhesive remains on the surface of the article because that surface remains covered by the cover tape.

The cover tape can be made of the same material or comprise the same material as the substrate (backing layer) or it can be made of a different material. Suitable cover tapes can include single- or multilayered films, coextruded films, laterally laminated films, and combinations thereof. Commercial tapes, such as commercial release tapes may be used, although the cover tapes may not require any release coating, release tapes contain. In a particular embodiment, the cover tape does not contain any release coating, in particular it does not contain any silicone, fluorochemical, or carbamate coating. Therefore, it is another advantage of the tabs as described herein that cheaper materials can be used because due to the absence or exposed adhesive there may be no need to for using tapes having expensive release coatings. In a preferred embodiment the cover tape is a unitary piece of thermoplastic film. In other embodiments, the cover tape can include multiple materials joined together. The cover tape may have the same thickness as the backing, or a greater or lower thickness. Typically, the cover tape may have a thickness between 100 and 5,000 µm. The cover tape typically has the same width than the substrate, but it may also have a larger or lower width than the underlying substrate.

The connection between substrate and cover tape is a bond created by ultrasonic welding, for example in form of welding dots. Therefore, cover tape and substrate are made of materials, or contain materials, that can be bonded together by ultrasonic welding. Preferably such materials are selected from the same or different thermoplastic materials and more preferably the same of different thermoplastic materials differing in melting points by less than 315,15 °K (42 °C).

The cover tape may cover all or only a portion of the middle region. It may also cover a portion of the fastening region adjacent to the middle region or it may cover the entire fastening region. The cover tape typically does not cover the second end region. Typically, the cover tape does not cover the outermost end of the first end region.

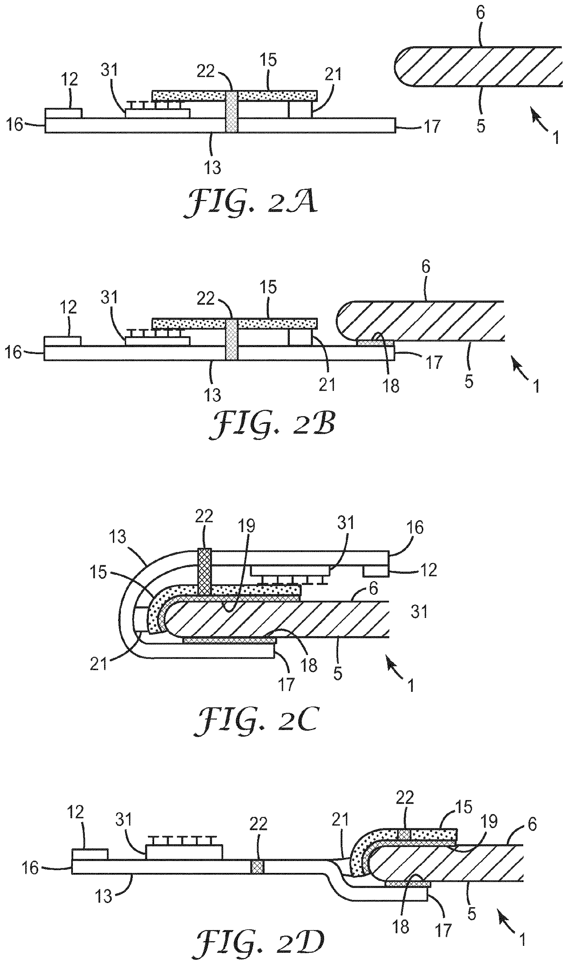

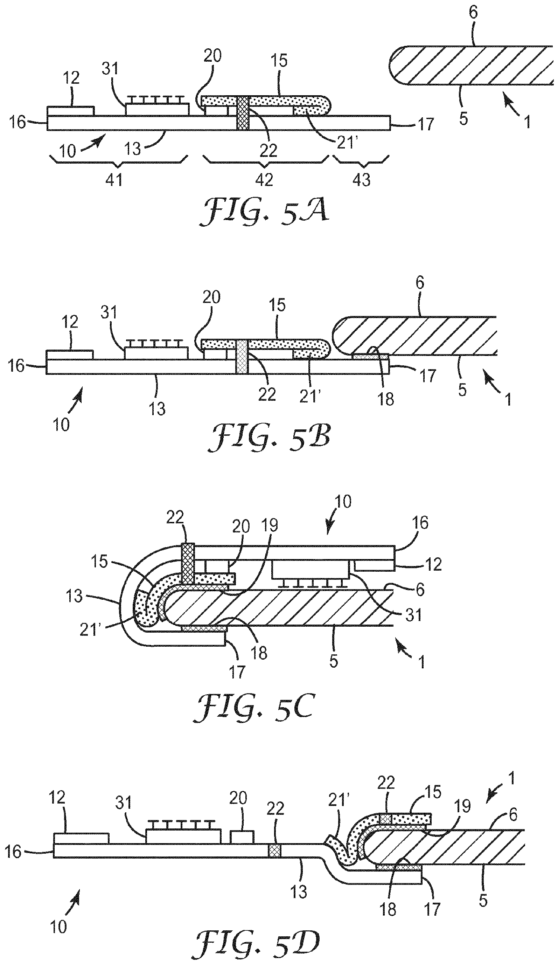

In one embodiment of the fastening tabs described herein, a second portion of the cover tape is connected to the substrate by at least one non-releasable connection, also referred to herein as "second connection" (represented in the figures as 21 and 21'). The second connection comprises a bond, also referred to herein as "second bond". The second connection is non-releasable. It is stronger than the releasable connection between the first portion of the cover tape and substrate and may be maintained when the releasable connection breaks and may be maintained at least during a single intended use of the article. The second connection may be a bond. When the tab is pulled away from its anti-flagging position the second bond may remain intact and may provide another connection of the substrate to the article - in addition to the connection of the tab to the article by the second end region of the tab. The second connection may not be as strong than the permanent bonds between fastening material and substrate or between second end region and the article, or it may be of equal strength, or even of greater strength. To create such a bond an adhesive or mixture of adhesives may be applied. Any suitable adhesive or mixtures of adhesives known in the art may be used. This second connection may be a direct connection, for example by an adhesive bond between the second portion of the cover tape and the substrate, or it may be an indirect bond, for example by an adhesive layer between substrate and second portion of the cover tape, for example by a double-sided adhesive tape or film. This means the tape or film contains adhesives on its opposite sides to create an adhesive connection between second portion of the cover tape and substrate. The second bond may also be a non-adhesive bond or a combination of adhesive and non-adhesive bonds. If present, the second connection of the cover tape is arranged in an area of the middle portion of the fastening tape that is in proximity to the second end portion, preferably it is arranged between the second end region and the releasable connection (22) of cover tape and substrate. In the embodiments shown in FIG. 1 and 2 the second connection between cover tape and substrate is an indirect connection. The second bond 21 between a second portion of the tape and the substrate is arranged in the middle region (42) between the releasable bond (22) and the second end region (43) of the tab and is provided by a double-sided adhesive film. In the embodiment shown in FIG 5 the second bond directly attaches a second portion of the cover tape to the substrate (21'). The second portion of the cover tape illustrated in FIG 5 is a folded portion of the cover tape. The direct bond can be provided by one or more adhesives or it can be created by means not using adhesives, for example welding, laminating, stiching etc. The second bond 21, 21' contains a strong bond to both the substrate and cover tape such that it assists in connecting the tab 10 via the second portion of the cover tape to the surface 5 of article 1 as shown in figures ID, 2D and 5D. This bond is stronger than the releasable, non-adhesive connection 22.

The middle region of the tabs according to the present disclosure may optionally contain one or more blanking films in the middle region, preferably in areas of the middle region covered by the cover tape. The blanking films (represented by 20 in figure 1) may be used to compensate for thickness differences along the length of the fastening tab, for example when the middle region covered by the cover tape is free of adhesive or contains large areas that are free of adhesives and thus may have a lower thickness in cross-section than the first end region. The blanking films can be attached to the substrate by a bond that is stronger than the bond by which the blanking films are connected to the cover tape - or the blanking films may not be bonded to the cover tape at all. When the cover tape is released from the tab the blanking film(s) remain on the substrate. The blanking films are optional. In one embodiment of the present disclsure, instead of using blanking films the substrate can be shaped to compensate for thickness differences, for example by the substrate having one or more raised areas in the middle region.
In the embodiment illustrated in FIG. 1A a blanking film 20 is used. The blanking film is only fixedly connected to the substrate 13 of the fastening tab 10 but not to the cover tape 15. Blanking film 20 helps to compensate for thickness differences along the length of the fastening tab 10 because the middle region covered by the cover tape may be free of exposed adhesive and thus may have a lower thickness in cross-section than the first end region 41. When the cover tape 15 is released, blanking film 20 remains on the substrate (compare FIG. 1 D). In the embodiment illustrated in FIG. 2 there may be no need for a blanking film 20 because the cover tape rests on the fastening material by which the thickness differences may be compensated sufficiently. In the embodiment illustrated in FIG 5, the cover tape is connected by one of its ends to the substrate and is then folded at least once to cover the middle region. By folding the cover tape, thickness differences may also be compensated. Instead of, or in addition to blanking films, the substrate may be shaped such that it contains in its middle region one or more raised areas that may compensate for a thickness difference between first end region and middle region (not shown in the figures).

FIGs 1B to 1D, 2B-D and 5B-D illustrate the application of the fastening tabs on the article. The article is illustrated as the ear of a diaper. The fastening tabs 10 are attached to the outer surface 5 of an ear 1 of a diaper by means of bond 18. The bond 18 may be an adhesive bond but can also be any other suitable connection for connecting the fastening tab 10 to the surface 5 including bonds created by ultrasonic welding, for example ultrasonic welding dots or seams, and lamination, stitching etc. In case an adhesive is used to create bond 18 the adhesive may be applied to the second end region 43 or to the surface 5 of the diaper's ear 1, or to both, before the tab is attached to the surface 5. After the tab 10 has been connected to the surface 5 via its maunfacturer's end 43 that fastening tab 10 is still in a flat configuration. In a subsequent step it will be folded around the diaper's ear 1 and attached to the inner surface 6 of the diaper's ear 1 to reach the anti-flagging position. The tab 10 is kept in that position by bond 19 to avoid flagging out of the first end region 41 of the fastening tab 10. FIGs 1C, 2C and 5C illustrates the fastening tab 10 in the folded-over configuration (anti flagging position), whereby additional connection between the cover tape 15 of the fastening tab 10 and the inner surface 6 of the article 1 is provided, by 19, for example an adhesive bond or any other suitable connection including ultrasonic welding dots bonding, heat welding seam, heat lamination etc. In case one or more adhesives are used to create the bond 19 the one or more adhesives may be applied to the outer surface of the cover tape 15 or to the appropriate area of the surface 6 of the article 1, or both, prior to folding the tab around the diaper's ear.

Because of the releasable, non-adhesive bond 22 and the bond 19, the fastening tab 10 is maintained in the folded-over configuration, which is called "anti-flagging" as outlined above. This is the desired configuration during manufacturing steps like folding and/or packaging of the article 1.

FIGs. ID, 2D and 5D show the fastening tabs 10 ready to use for fastening. The fastening tabs 10 are typically grasped by a user by their outermost ends 16 of the first end region 41 and pulled open from the anti-flagging position, thus breaking the releasable, non-adhesive bond 22 and bringing the fastening tab 10 into an (intermediate) flat configuration. Then the tab is folded again to engage the fastening elements of its fastening material 31 with the appropriate receiving surface on the article, e.g. diaper the backsheet or a landing zone on a diaper (not shown in the figures). It is to be noted, that cover tape 15 stays connected to the surface of the article as illustrated in Fig. 1D, 2D and 5D.

In one aspect of the present disclosure there is provided a method of applying a fastening tab an article, the method comprising
- providing a fastening tab as described herein;
- attaching the fastening tab with its second end region to a first surface of the article.
At least one adhesive is applied to the second end region of the tab or to the surface of the article before step b is carried out. The method further comprises attaching a surface of the cover tape release layer that face away from the substrate to a second surface of the article by folding the tab around the first surface and bringing the tab into an anti-flagging position on the article. The second surface of the article preferably is the opposite side of the first surface of that article. Before attaching the surface of the cover tape to the second surface of the article, an adhesive 1may be applied, either to the surface of the cover tape or to the second surface of the article or both. Instead of or in addition to using adhesives the cover tape can be attached to the second surface of the article by a bonding method that does not employ adhesives, including but not limited to laminating, ultrasonic welding and stitching.

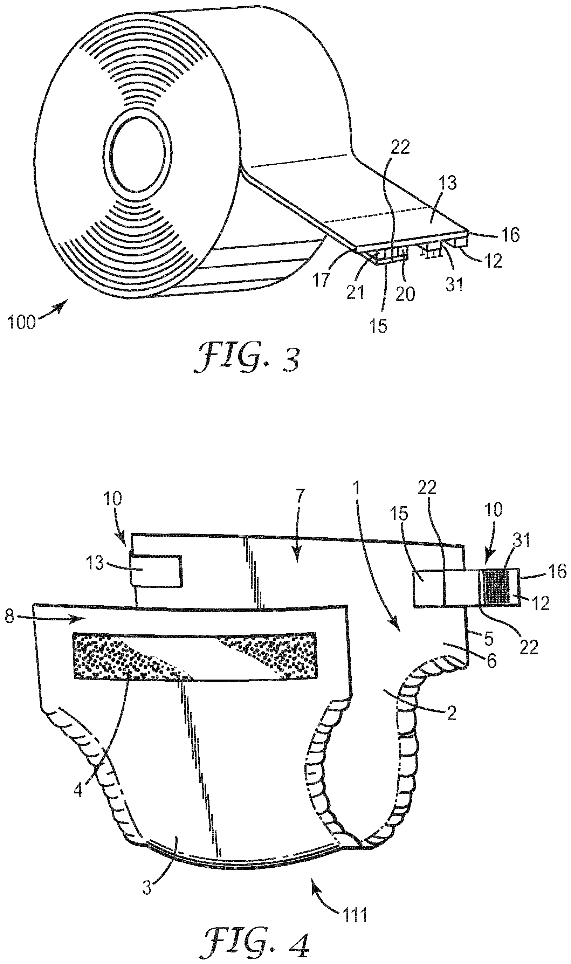

Although a diaper has been used for illustration in the figures, it is understood that other articles may be used also. Suitable articles include disposable articles including but not limited to wrap-around films, wrap-around fabrics, medical garments, gloves, bags. A typical class of articles includes disposable hygienic article like sanitary napkins, incontinence pads and diapers. Figure 4 provides a schematic, perspective view of a diaper with two fastening tabs according to the present disclosure, the tabs are shown viewed from the top. The tab 10 shown on the left ear of the diaper is in the anti-flagging position (e.g. a position as shown in figures 1C, 2C and 5C. The tab 10 shown on the right ear of the diaper (1) corresponds to the position shown in figures ID, 2D and 5D, i.e. a position after release from the anti-flagging position and ready for engagement with a receiving surface. Referring to Fig. 4, a conventional diaper construction 111 is shown comprising a liquid permeable topsheet 2 and a liquid impermeable backsheet 3. Between the topsheet 2 and the backsheet 3 would be an absorbent core (not shown). The liquid permeable topsheet 2 would generally be a nonwoven web or like permeable structure which would allow the passage of liquids to the absorbent core. The liquid impermeable backsheet 3 conventionally is a film or a laminate of a film with a nonwoven. The backsheet film is generally a polyolefin thermoplastic film, which can be provided with breathability. The non-woven material laminated to such a film would be on the outer surface of the diaper 111 to provide a soft texture and cloth-like feel. The nonwoven can also provide a surface that could allow a hook material to engage, in certain circumstances, to allow for closure attachment and/or disposability of the diaper 111 following use. The liquid permeable topsheet would comprise a conventional material known for this use including spunbond webs, melt blown webs, carded webs, and the like. These webs are conventionally formed of thermoplastic and hydrophobic polymer fibers, such as polyolefin or polyester fibers.

The backsheet 3 is a generally liquid impermeable web, optionally an air permeable web or film and conventionally uses a thin polyolefin polymer film generally less than 1 to 2 mils (25,4 to 50,8 µm) thick. For use in a mechanical fastener closure system, the backsheet 3 is preferably a laminate of a substantially liquid impermeable film with a woven, nonwoven, knitted or stitch bonded or like fibrous web, which fibrous web is suitably engageable with the mechanical fastener material 31 on the fastening tab 10. Adhesive lamination, pattern heat bonding or extrusion lamination of the web with the liquid impermeable film can form this laminate.

The loop material can be any conventional loop fabric such as disclosed, for example, in U.S. Patent Nos. 5,176,670 or 5,256,231. The loop fabric can be a conventional fibrous structure such as a nonwoven, a knitted material, a stitch bonded material or the like, formed of natural or synthetic fibers, which fibers can be either continuous or discontinuous and/or bonded. Generally, the loop material must have sufficient available fibers and loft to allow penetration of the mechanical fastening material 31 into the loop structure but still have enough fibers available to engage with the individual hooks.

The fastening tabs according to the present disclosure can be produced, for example, by providing a web of fastening material and joining the web of fastening material with a substrate layer. The cover tape may be applied and releasably connected to the substrate prior to or simultaneously with the fastening material. Optional features like fingerlift, blanking films may be applied to the substrate after or before the cover tape has been connected to the middle region of the substrate by the releasable connection. The optional non-releasable connection between substrate and cover tape may be applied before, after or simultaneously with the releasable connection of the cover tape to the substrate. This way an "endless" web of fastening tabs, a so-called fastening tabs tape can be prepared. The "endless" tapes may be provided in a roll form as is generally shown in FIG. 3. This roll 100 shown in FIG. 3 contains a plurality of fastening tabs 10 having a finger lift 12, a fastening region with fastening material 31 comprising hooks and a release layer 15. Desirably the fastening tabs are arranged in endless form on the tape so that individual tabs can be cut from the fastening tape (as shown in FIG. 3). Alternatively, the fastening tabs may be arranged individually on the tape for example spaced from each other along the machine direction. Individual tab elements 10 can be cut from the roll and attached to an article.

The same reference numbers used in the figures denote the same items.

## Claims

1. A fastening tab (10) for an article (111) comprising a substrate (13) having a first end region (41) with a first outermost end (16) and opposite thereto a second end region (43) with a second outermost end (17), and a middle region between the first and second end regions (41, 43), wherein the first end region (41) comprises at least one fastening region comprising a fastening material (31) attached to the substrate (13) wherein the fastening material (31) comprises a plurality of mechanical fastening elements for releasable fastening, the tab (10) further comprising a cover tape (15) covering at least a portion of the middle region (42), and wherein at least a first portion of the cover tape (15) is connected to the middle region (42) by at least one releasable connection (22), wherein the at least one releasable connection (22) comprises a releasable, non-adhesive bond (22), wherein the cover tape (15) is connected by a second portion to the substrate (13) by at least one non-releasable connection (21), wherein the at least one non-releasable connection (21) is situated between the at least one releasable connection (22) and the second end region (43) and wherein the cover tape (15) comprises a surface that can be attached to the article (111) to create a bond between the article (111) and that surface of the cover tape (15), **characterized in that** the releasable, non-adhesive bond (22) is created by ultrasonic welding and wherein the tape (15) and the substrate (13) comprise, at least at their releasable connection (22), a thermoplastic material that does not differ in melting point or differs in melting point by less than (315,15 °K) 42°C.

2. The fastening tab (10) according to claim 1, wherein the middle region (42), preferably the middle region (42) covered by the tape (15), comprises at least one area that is free of exposed adhesive, preferably the major portion of the middle region (42) is free of exposed adhesive.

3. The fastening tab (10) according to any one of claims 1 or 2, wherein the middle region (43) comprises one or more blanking films (20) between substrate (13) and cover tape (15).

4. The fastening tab (10) according to any one of claims 1 to 3, wherein the tape covers (15) at least a part of the fastening region.

5. The fastening tab (10) according to any one of claims 1 to 4, wherein the tape (15) does not cover the second end region (43).

6. The fastening tab (10) according to any one of claims 1 to 5, wherein at the first portion of the tape (15) is not connected to the substrate (13) by an adhesive.

7. A tape comprising a plurality of fastening tabs (10) according to any one of claims 1 to 6, wherein the fastening tabs (10) are arranged either individually or in endless form on the tape in a cross section so that individual tabs (10) can be cut from the fastening tape.

8. The tape according to claim 7 wound up into a roll (100).

9. An article (111) comprising a fastening tab (10) according to any one of claims 1 to 6, wherein the tab (10) is connected to a surface (1, 5, 6) of the article (111) by the second end portion (43) of the tab (10).

10. Method of making a fastening tab (10) according to any of claims 1 to 6, the method comprising the steps of
a) providing the cover tape (15) and the substrate (13) having the first end region (41) with a first outermost end (16) and opposite thereto the second end region (43) with a second outermost end (17), and the middle region (42) between the first and second regions (41, 43), wherein the first end region (41) comprises at least one fastening region comprising a fastening material (31) attached to the substrate (13) wherein the fastening material (31) comprises a plurality of mechanical fastening elements for releasable fastening;
b) connecting the cover tape (15) at least by its first portion to the middle region (42) of the substrate (13) by at least one releasable connection (22) made by ultrasonic welding such that the tape (15) covers at least a portion of the middle region (42) of the substrate (13), wherein the at least one releasable connection (22) comprises a releasable, non-adhesive bond (22).

11. Method of applying a fastening tab (10) to an article (111), the method comprising
i) providing a fastening tab (10) according to any one of claims 1 to 6;
ii) attaching the fastening tab (10) with its second end region (43) to a surface (1, 5, 6) of the article (111).

12. The method according to claim 11, wherein at least one adhesive is applied to the second end region (43) of the tab (10) or to the surface (1, 5, 6) of the article (111) before carrying out step ii).

## Patentansprüche

1. Befestigungslasche (10) für einen Artikel (111), umfassend ein Substrat (13), das eine erste Endregion (41) mit einem ersten äußersten Ende (16) und gegenüberliegend dazu eine zweite Endregion (43) mit einem zweiten äußersten Ende (17) und eine mittlere Region zwischen der ersten und der zweiten Endregion (41, 43) aufweist, wobei die erste Endregion (41) mindestens eine Befestigungsregion umfasst, die ein Befestigungsmaterial (31) umfasst, das am Substrat (13) angebracht ist, wobei das Befestigungsmaterial (31) eine Vielzahl von mechanischen Befestigungselementen zum lösbaren Befestigen umfasst, wobei die Lasche (10) ferner ein Abdeckband (15) umfasst, das mindestens einen Abschnitt der mittleren Region (42) abdeckt, und wobei mindestens ein erster Abschnitt des Abdeckbandes (15) mit der mittleren Region (42) durch mindestens eine lösbare Verbindung (22) verbunden ist, wobei die mindestens eine lösbare Verbindung (22) eine lösbare, nicht klebende Bindung (22) umfasst, wobei das Abdeckband (15) durch einen zweiten Abschnitt mit dem Substrat (13) durch mindestens eine nicht lösbare Verbindung (21) verbunden ist, wobei sich die mindestens eine nicht lösbare Verbindung (21) zwischen der mindestens einen lösbaren Verbindung (22) und der zweiten Endregion (43) befindet und wobei das Abdeckband (15) eine Oberfläche umfasst, die an dem Artikel (111) angebracht werden kann, um eine Bindung zwischen dem Artikel (111) und dieser Oberfläche des Abdeckbandes (15) herzustellen, **dadurch gekennzeichnet, dass** die lösbare, nicht klebende Bindung (22) durch Ultraschallschweißen erzeugt wird und wobei das Band (15) und das Substrat (13) mindestens an ihrer lösbaren Verbindung (22) ein thermoplastisches Material aufweisen, das sich im Schmelzpunkt nicht oder um weniger als (315,15 °K) 42 °C unterscheidet.

2. Befestigungslasche (10) nach Anspruch 1, wobei die mittlere Region (42), vorzugsweise die mittlere Region (42), die durch das Band (15) abgedeckt ist, mindestens einen Bereich aufweist, der frei von freiliegendem Klebstoff ist, wobei vorzugsweise der größte Abschnitt der mittleren Region (42) frei von freiliegendem Klebstoff ist.

3. Befestigungslasche (10) nach einem der Ansprüche 1 oder 2, wobei die mittlere Region (43) einen oder mehrere Abdeckfilme (20) zwischen Substrat (13) und Abdeckband (15) umfasst.

4. Befestigungslasche (10) nach einem der Ansprüche 1 bis 3, wobei das Band (15) mindestens einen Teil der Befestigungsregion abdeckt.

5. Befestigungslasche (10) nach einem der Ansprüche 1 bis 4, wobei das Band (15) die zweite Endregion (43) nicht abdeckt.

6. Befestigungslasche (10) nach einem der Ansprüche 1 bis 5, wobei der erste Abschnitt des Bandes (15) nicht durch einen Klebstoff mit dem Substrat (13) verbunden ist.

7. Band, umfassend eine Vielzahl von Befestigungslaschen (10) nach einem der Ansprüche 1 bis 6, wobei die Befestigungslaschen (10) in einem Querschnitt auf dem Band einzeln oder endlos so angeordnet sind, dass einzelne Laschen (10) vom Befestigungsband abgeschnitten werden können.

8. Band nach Anspruch 7, das zu einer Rolle (100) aufgewickelt ist.

9. Artikel (111), umfassend eine Befestigungslasche (10) nach einem der Ansprüche 1 bis 6, wobei die Lasche (10) mit einer Oberfläche (1, 5, 6) des Artikels (111) durch die zweite Endregion (43) der Lasche (10) verbunden ist.

10. Verfahren zum Herstellen einer Befestigungslasche (10) nach einem der Ansprüche 1 bis 6, wobei das Verfahren die Schritte umfasst
a) Bereitstellen des Abdeckbandes (15) und des Substrats (13), das die erste Endregion (41) mit einem ersten äußersten Ende (16) und gegenüberliegend dazu die zweite Endregion (43) mit einem zweiten äußersten Ende (17) und die mittlere Region (42) zwischen der ersten und der zweiten Region (41, 43) aufweist, wobei die erste Endregion (41) mindestens eine Befestigungsregion umfasst, die ein Befestigungsmaterial (31) umfasst, das an dem Substrat (13) angebracht ist, wobei das Befestigungsmaterial (31) eine Vielzahl von mechanischen Befestigungselementen zum lösbaren Befestigen umfasst;
b) Verbinden des Abdeckbandes (15) mindestens durch seinen ersten Abschnitt mit der mittleren Region (42) des Substrats (13) durch mindestens eine lösbare Verbindung (22), die durch Ultraschallschweißen hergestellt wird, sodass das Band (15) mindestens einen Abschnitt der mittleren Region (42) des Substrats (13) abdeckt, wobei die mindestens eine lösbare Verbindung (22) eine lösbare, nicht klebende Bindung (22) umfasst.

11. Verfahren zum Aufbringen einer Befestigungslasche (10) auf einen Artikel (111), wobei das Verfahren umfasst
i) Bereitstellen einer Befestigungslasche (10) nach einem der Ansprüche 1 bis 6;
ii) Befestigen der Befestigungslasche (10) mit ihrer zweiten Endregion (43) auf einer Oberfläche (1, 5, 6) des Artikels (111).

12. Verfahren nach Anspruch 11, wobei mindestens ein Klebstoff auf die zweite Endregion (43) der Lasche (10) oder auf die Oberfläche (1, 5, 6) des Artikels (111) vor dem Durchführen von Schritt ii) aufgebracht wird.

## Revendications

1. Languette de fixation (10) pour un article (111) comprenant un substrat (13) possédant une première région d'extrémité (41) avec une première extrémité extérieure (16) et opposée à celle-ci, une deuxième région d'extrémité (43) avec une deuxième extrémité extérieure (17), et une région centrale entre les première et deuxième régions d'extrémité (41, 43), dans laquelle la première région d'extrémité (41) comprend au moins une région de fixation comprenant un matériau de fixation (31) fixé au substrat (13) dans laquelle le matériau de fixation (31) comprend une pluralité d'éléments de fixation mécanique pour une fixation détachable, la languette (10) comprenant en outre un ruban de couverture (15) couvrant au moins une partie de la région centrale (42), et dans laquelle au moins une première partie de la bande de couverture (15) est reliée à la région centrale (42) par au moins une connexion détachable (22), dans laquelle l'au moins une connexion détachable (22) comprend une liaison non adhésive,
détachable (22), dans laquelle le ruban de couverture (15) est connecté par une deuxième partie au substrat (13) par au moins une connexion non détachable (21), dans laquelle l'au moins une connexion non détachable (21) est située entre l'au moins une connexion détachable (22) et la deuxième région d'extrémité (43) et dans laquelle le ruban de couverture (15) comprend une surface qui peut être fixée à l'article (111) de façon à créer une liaison entre l'article (111) et la surface du ruban de couverture (15), **caractérisée en ce que** la liaison non adhésive détachable (22) est créée par soudage par ultrasons et dans laquelle le ruban (15) et le substrat (13) comprennent, au moins au niveau de leur connexion détachable (22), un matériau thermoplastique, qui ne diffèrent pas au niveau du point de fusion ou qui diffèrent de moins de (315,15 °K) 42 °C concernant le point de fusion.

2. Languette de fixation (10) selon la revendication 1, dans laquelle la région centrale (42), de préférence la région centrale (42) couverte par le ruban (15), comprend au moins une zone qui est exempte d'adhésif exposé, de préférence la majeure partie de la région centrale (42) est exempte d'adhésif exposé.

3. Languette de fixation (10) selon l'une quelconque des revendications 1 ou 2, dans laquelle la région centrale (43) comprend un ou plusieurs films protecteurs (20) entre un substrat (13) et un ruban de couverture (15).

4. Languette de fixation (10) selon l'une quelconque des revendications 1 à 3, dans laquelle le ruban (15) couvre au moins une partie de la région de fixation.

5. Languette de fixation (10) selon l'une quelconque des revendications 1 à 4, dans laquelle le ruban (15) ne couvre pas la deuxième région d'extrémité (43).

6. Languette de fixation (10) selon l'une quelconque des revendications 1 à 5, dans laquelle la première partie du ruban (15) n'est pas connectée au substrat (13) au moyen d'un adhésif.

7. Ruban comprenant une pluralité de languettes de fixation (10) selon l'une quelconque des revendications 1 à 6, dans lequel les languettes de fixation (10) sont agencées soit individuellement, soit sous une forme sans fin sur le ruban dans une section transversale de sorte que des languettes individuelles (10) peuvent être coupées du ruban de fixation.

8. Ruban selon la revendication 7, enroulé en un rouleau (100).

9. Article (111) comprenant une languette de fixation (10) selon l'une quelconque des revendications 1 à 6, dans lequel la languette (10) est reliée à une surface (1, 5, 6) de l'article (111) par la deuxième partie d'extrémité (43) de la languette (10).

10. Procédé de fabrication d'une languette de fixation (10) selon l'une quelconque des revendications 1 à 6, le procédé comprenant les étapes consistant à
a) fournir le ruban de couverture (15) et le substrat (13) ayant la première région d'extrémité (41) avec une première extrémité extérieure (16) et opposée à celle-ci, la deuxième région d'extrémité (43) avec une deuxième extrémité extérieure (17), et la région centrale (42) entre les première et deuxième régions (41, 43), dans lequel la première région d'extrémité (41) comprend au moins une région de fixation comprenant un matériau de fixation (31) fixé au substrat (13) dans lequel le matériau de fixation (31) comprend une pluralité d'éléments de fixation mécanique pour une fixation de manière détachable ;
b) connecter le ruban de couverture (15) au moins par sa première partie à la région centrale (42) du substrat (13) par au moins une connexion détachable (22) faite par soudage par ultrasons de telle sorte que le ruban (15) couvre au moins une partie de la région centrale (42) du substrat (13), dans lequel l'au moins une connexion détachable (22) comprend une liaison non adhésive, détachable (22).

11. Procédé d'application d'une languette de fixation (10) sur un article (111), le procédé comprenant
i) la fourniture d'au moins une languette de fixation (10) selon l'une quelconque des revendications 1 à 6 ;
ii) la fixation de la languette de fixation (10) avec sa deuxième région d'extrémité (43) sur une surface (1, 5, 6) de l'article (111).

12. Procédé selon la revendication 11, dans lequel au moins un adhésif est appliqué sur la deuxième région d'extrémité (43) de la languette (10) ou sur la surface (1, 5, 6) de l'article (111) avant de réaliser l'étape ii).
